(19) Europäisches Patentamt
European Patent Office
Office européen des brevets

(11) Veröffentlichungsnummer : **0 064 702**
**B1**

(12) # EUROPÄISCHE PATENTSCHRIFT

(45) Veröffentlichungstag der Patentschrift : **02.05.84**

(51) Int. Cl.³ : **C 07 C 93/14**

(21) Anmeldenummer : **82103737.1**

(22) Anmeldetag : **03.05.82**

(54) **4-tert.-Butoxybenzylamine.**

(30) Priorität : **11.05.81 DE 3118628**

(43) Veröffentlichungstag der Anmeldung : **17.11.82 Patentblatt 82/46**

(45) Bekanntmachung des Hinweises auf die Patenterteilung : **02.05.84 Patentblatt 84/18**

(84) Benannte Vertragsstaaten : **CH DE FR GB IT LI**

(56) Entgegenhaltungen :
**FR-A- 1 096 323**
**US-A- 2 676 173**

(73) Patentinhaber : **BASF Aktiengesellschaft**
**Carl-Bosch-Strasse 38**
**D-6700 Ludwigshafen (DE)**

(72) Erfinder : **Barl, Manfred, Dr.**
**Pappelstrasse 2**
**D-6701 Otterstadt (DE)**
Erfinder : **Degner, Dieter, Dr.**
**Kurpfalzstrasse 8**
**D-6701 Dannstadt-Schauernheim (DE)**
Erfinder : **Siegel, Hardo, Dr.**
**Hans-Purrmann-Allee 25**
**D-6720 Speyer (DE)**

**EP 0 064 702 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

4-tert.-Butoxybenzylamine

Die vorliegende Erfindung betrifft neue 4-tert.-Butoxybenzylamine, deren Ammoniumsalze und ein Verfahren zur Herstellung der 4-tert.-Butoxybenzylammoniumsalze.

4-tert.-Butoxybenzylamine und ihre Ammoniumsalze sind neue Verbindungen, mit denen die Aufgabe, 4-Hydroxybenzylamine und deren Folgeprodukte herzustellen, besonders leicht gelöst werden kann und die deshalb erhebliche technische Bedeutung besitzen.

Es wurde gefunden, daß 4-tert.-Butoxybenzylamine und deren Ammoniumsalze der allgemeinen Formel (I)

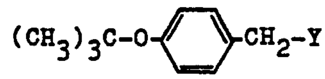

(I)

wobei Y für die Reste

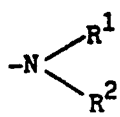

oder

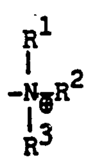

$X^{\ominus}$

steht, in denen $R^1$ und $R^2$ ein Wasserstoffatom, eine Alkyl-, Cycloalkyl-, Aralkyl- oder Arylgruppe mit 1 bis 15 Kohlenstoffatomen, $R^3$ ein Wasserstoffatom, eine Alkyl-, Cycloalkyl oder Aralkylgruppe mit 1 bis 15 Kohlenstoffatomen und $X^{\ominus}$ ein Hydroxylion oder ein Säureanion bedeutet, bequem hergestellt werden können durch Umsetzung des aus der DE-OS 29 35 398 bekannten 4-tert.-Butoxybenzaldehyds mit Ammoniak, einem primären oder sekundären Amin der Formel $R^1$—NH—$R^2$ und Wasserstoff in Gegenwart eines Hydrierkatalysators (hydrierende Aminierung), an die sich gegebenenfalls die Protonierung bzw. Alkylierung zu den entsprechenden Ammoniumverbindungen anschließt. Die Reste $R^1$ und/oder $R^2$ können beispielsweise ein Wasserstoffatom, eine Methyl-, Ethyl-, Isopropyl-, Butyl-, Hexyl-, 2-Ethyl-hexyl-, Decyl-, Dodecyl-, Pentadecyl-, Cyclopentyl-, Cyclohexyl-, 4-Methylcyclohexyl-, Benzyl-, Phenyl- oder p-Chlorphenylgruppe bedeuten.

Der durch Protonierung bzw. Alkylierung eingeführte Rest $R^3$ hat, mit Ausnahme der Arylgruppen, die Bedeutung von $R^1$ und $R^2$.

$X^{\ominus}$ bedeutet ein Hydroxylion oder ein Säureanion, z. B. ein Fluorid-, Chlorid-, Bromid-, Iodid-, Hydrogensulfat-, Perchlorat-, Methosulfat-, p-Toluolsulfonat-, Acetat- oder Trifluoracetatanion.

Im einzelnen werden die erfindungsgemäßen Schritte wie folgt durchgeführt :

Die hydrierende Aminierung wird in der Regel bei erhöhter Temperatur (60 bis 200 °C) und i. a. erhöhtem Druck (z. B. 50 bis 250 bar) vorgenommen. Als Katalysatoren kommen vor allem Übergangsmetalle der 8. Nebengruppe in Frage, besonders Kobalt, Nickel, Palladium, Platin aber auch Kupfer und die unter dem Begriff « Adkins-Katalysatoren » bekannten Katalysatoren auf der Grundlage von Kupfer und Chrom.

Als besonders vorteilhaft gilt die Umsetzung mit Ammoniak in Gegenwart von Kobalt oder Nickel enthaltenden Katalysatoren, während Umsetzungen mit primären und sekundären Aminen oft in Gegenwart von Palladium oder Platin vorgenommen werden. Die Katalysatoren können als Trägerkatalysatoren auf z. B. Kielselsäure, Aluminiumoxid oder Aktivkohle verwendet werden oder trägerfrei. Sie können mit anderen Metallen oder Nichtmetallen als Aktivatoren versehen sein.

Die Protonierung bzw. Alkylierung zu den Ammoniumsalzen wird mit konzentrierten oder verdünnten Säuren bzw. mit Alkylierungsmitteln vorgenommen. Als Reagentien können dafür z. B. Halogenwasserstoffsäuren, Schwefelsäure, Perchlorsäure, p-Toluolsulfonsäure, Essigsäure, Trifluoressigsäure bzw. Alkylsulfate, Alkyltosylate, Alkyl- oder Aralkylhalogenide oder Trialkyloxoniumsalze eingesetzt werden. Dabei führt man die Protonierung bzw. Alkylierung unter den an sich bekannten Synthesebedingungen für Ammoniumsalze durch.

Alle Umsetzungen können absatzweise oder fortlaufend gestaltet werden, wobei jeweils fachübliche Vorrichtungen bzw. Anordnungen verwendet werden.

Da die tert.-Butylgruppe bei milden Bedingungen (z. B. mit verdünnter Schwefelsäure) verseift werden kann, sind die Verbindungen als O-geschützte Aminophenole zu betrachten. Sie sind damit wertvolle Zwischenprodukte bei der Synthese von Farbstoffen, Pharmazeutika und Pflanzenschutzmitteln. Z. B.

kann Tyramin ausgehend von tert.-Butoxybenzylamin durch Ersatz der Aminogruppe gegen die Nitrilgruppe und Hydrierung leicht erhalten werden.

Die in den nachstehenden Beispielen im Labormaßstab beschriebenen Umsetzungen können von jedem Fachmann leicht in technischem Maßstab ausgeführt werden; für manche, gewerblich nur für spezielle Zwecke benötigte Amine bzw. Ammoniumsalze ist auch deren Synthese im Labormaßstab bereits ausreichend.

## Beispiel 1

Herstellung von 4-tert.-butoxybenzylamin

In einem 5 l-Autoklaven mit Hubrührer werden 500 g Ammoniak und 50 g Raney-Kobalt vorgelegt. Man preßt 10 bar Wasserstoff auf, bringt auf 140 °C, erhöht den Wasserstoffdruck auf 250 bar und pumpt dann innerhalb von 4 Stunden 800 g 4-tert.-Butoxybenzaldehyd zu. Der verbrauchte Wasserstoff wird stündlich ersetzt. Man läßt 3 Stunden nachreagieren. Nach der fraktionierten Destillation erhält man 699 g 4-tert.-Butoxybenzylamin (Sdp. 146 bis 148 °C/32 mbar, rechnerische Ausbeute 87 %).

$^1$H-NMR($CDCl_3$) : δ = 7,15 ppm (d, 2 H) ; 6,9 ppm (d, 2 H) ;
3,75 ppm (s, 2 H) ; 1,5 ppm (s, 2 H, $NH_2$) ;
1,3 ppm (s, 9 H)

## Beispiel 2

Herstellung von N-Methyl-4-tert.-butoxybenzylamin

In einem 5 l-Autoklaven werden 70 g eines Katalysators, der 0,4 % Palladium, 5 % Silber und 1 % Mangan auf Kieselsäure enthält, und 1 kg Monomethylamin vorgelegt. Man preßt 20 bar Wasserstoff auf, heizt auf 180 °C und erhöht den Wasserstoffdruck auf 250 bar. Dann wird innerhalb von 5 Stunden 1 kg 4-tert.-Butoxybenzaldehyd zugepumpt. Der verbrauchte Wasserstoff wird stündlich ersetzt. Nach der fraktionierten Destillation erhält man 848 g N-Methyl-4-tert.-butoxybenzylamin (Sdp. 114 °C/3 mbar, Ausbeute 82 %).

$^1$H-NMR ($CDCl_3$) : δ = 7,1 ppm (d, 2 H) ; 6,85 ppm (d, 2 H) ;
3,6 ppm (s, 2 H) ; 2,35 ppm (s, 3 H, $-CH_2-$ + $-NH-$) ;
1,3 ppm (s, 9 H).

## Beispiel 3

Herstellung von N,N-Dimethyl-4-tert.-butoxybenzylamin

In einem 5 l-Autoklaven werden 1 350 g Dimethylamin und 100 g des in Beispiel 2 angegebenen Katalysators vorgelegt. Man verfährt bei 140 °C wie vorstehend angegeben und pumpt 1 780 g 4-tert.-Butoxybenzaldehyd innerhalb von 5 Stunden zu. Man ersetzt stündlich den verbrauchten Wasserstoff und läßt 8 Stunden nachreagieren.

Die gaschromatographische Analyse zeigt, daß der Umsatz vollständig ist. Die Ausbeute (Selektivität) bezogen auf Aldehyd beträgt 95 %. Durch fraktionierte Destillation erhält man aus dem rohen Reaktionsgemisch 1 778 g N,N-Dimethyl-4-tert.-butoxybenzylamin (Sdp. 142 bis 144 °C/33,5 mbar).

$^1$H-NMR ($CDCl_3$) : 7,15 ppm (d, 2 H) ; 6,9 ppm (d, 2 H) ;
3,35 ppm (s, 2 H) ; 2,2 ppm (s, 6 H) ;
1,35 ppm (s, 9 H).

## Beispiel 4

Herstellung von N-n-Butyl-4-tert.-butoxybenzylamin

In einem 300 ml fassenden Autoklaven mit Begasungsrührer werden 50 g n-Butylamin, 60 g 4-tert.-Butoxybenzaldehyd sowie 5 g des vorstehend beschriebenen Palladiumkatalysators 11 Stunden lang bei 200 °C und 300 bar mit Wasserstoff umgesetzt. Die gaschromatographische Analyse zeigt vollständigen Umsatz und eine Selektivität von 92 %. Durch Destillation (Sdp. 126 bis 128 °C/0,5 mbar) erhält man 81 g N-n-Butyl-4-tert.-butoxybenzylamin.

$^1$H-NMR ($CDCl_3$) : δ = 7,2 ppm (d, 2 H) ; 6,9 ppm (d, 2 H) ;
3,7 ppm (s, 2 H) ; 2,6 ppm (t, 2 H) ;
1,1 bis 1,6 ppm (m, 4 H + NH) ; 1,35 ppm (s, 9 H) ;
0,9 ppm (t, 3 H).

Beispiel 5

Herstellung von N,N,N-Trimethyl-4-tert.-butoxybenzylammoniummethosulfat

In einem 1 l fassenden Rührkolben werden 207 g N,N-Dimethyl-4-tert.-butoxybenzylamin zu einer Lösung von 126 g Dimethylsulfat in 500 ml Acetonitril bei 80 °C innerhalb von 30 Minuten zugesetzt. Man läßt eine Stunde bei 80 °C nachreagieren. Man entfernt das Lösungsmittel bei 60 °C/30 mbar in einem Rotationsverdampfer. Der Rückstand (327 g) enthält analysenreines N,N,N-Trimethyl-4-tert.-butoxybenzylammonium-methosulfat (Fp : 127 bis 129 °C), als äußerlich amorphes leicht gelbliches Pulver.

$^1$H-NMR ($CDCl_3$) : $\delta$ = 7,5 ppm (d, 2 H) ; 7,0 ppm (d, 2 H) ;
4,65 ppm (s, 2 H) ; 3,7 ppm (s, 3 H) ;
3,2 ppm (s, 9 H) ; 1,35 ppm (s, 9 H).

Beispiel 6

Herstellung von N-Cyclohexyl-4-tert.-butoxybenzylamin

In einem 2,5 l-Autoklaven mit Hubrührer werden 495 g Cyclohexylamin und 100 g des in Beispiel 2 verwendeten Katalysators vorgelegt. Man preßt 20 bar Wasserstoff auf, bringt auf 180 °C und preßt Wasserstoff bis zu einem Gesamtdruck von 200 bar nach. Dann werden innerhalb von 3 Stunden 445 g 4-tert.-Butoxybenzaldehyd angesetzt. Man ersetzt den verbrauchten Wasserstoff und läßt 30 Minuten nachreagieren. Durch fraktionierte Destillation erhält man 466 g N-Cyclohexyl-4-tert.-butoxybenzylamin vom Sdp. 160 bis 164 °C/0,6 mbar entsprechend einer Ausbeute von 72 %.

$^1$H-NMR ($CDCl_3$) : $\delta$ = 7,2 ppm (d, 2 H) ; 6,9 ppm (d, 2 H) ;
3,75 ppm (s, 2 H) ; 2,5 ppm (m, 1 H) ;
1,5 bis 2,3 ppm (m, 10 H) ;
1,35 ppm (s, 9 H) ; 1,2 ppm (s, NH)

Beispiel 7

Herstellung von N-Phenyl-4-tert.-butoxybenzylamin

In einem 5 l-Autoklaven mit Hubrührer werden 930 g Anilin sowie 200 g des in Beispiel 2 verwendeten Katalysators vorgelegt. Man preßt 20 bar Wasserstoff auf, bringt auf 200 °C und preßt bis zu einem Gesamtdruck von 200 bar Wasserstoff nach. Innerhalb von 3 Stunden werden 890 g 4-tert.-Butoxybenzaldehyd zugesetzt. Man ergänzt den verbrauchten Wasserstoff und läßt 1 Stunde nachreagieren. Durch fraktionierte Destillation erhält man 974 g N-Phenyl-4-tert.-butoxybenzylamin vom Sdp. 158 bis 163 °C/0,6 mbar entsprechend einer Ausbeute von 77 %.

$^1$H-NMR ($CDCl_3$) : $\delta$ = 7,25 ppm (d, 2 H) ; 7,1 ppm (t, 2 H) ;
6,95 ppm (d, 2 H) ; 6,7 ppm (d, 1 H) ;
6,6 ppm (d, 2 H) ; 4,2 ppm (s, 2 H) ;
3,9 ppm (s, NH) ; 1,35 ppm (s, 9 H).

Beispiel 8

Herstellung von N-Dodecyl-4-tert.-butoxybenzylamin

In einem 300 ml-Autoklaven mit Begasungsrührer werden 50 g 4-tert.-Butoxybenzaldehyd, 100 g Dodecylamin sowie 5 g des in Beispiel 2 verwendeten Katalysators 12 Stunden lang bei 200 °C mit 250 bar Wasserstoff behandelt. Der Druckabfall beträgt insgesamt 185 bar ; der jeweils verbrauchte Wasserstoff wird stündlich ersetzt. Durch Destillation (Sdp. 168 bis 173 °C/0,3 mbar) erhält man 71 g N-Dodecyl-4-tert.-Butoxybenzylamin (Ausbeute : 74 %).

$^1$H-NMR ($CDCl_3$) : $\delta$ = 7,25 ppm (d, 2 H) ; 6,95 ppm (d, 2 H) ;
3,7 ppm (s, 2 H) ; 2,65 ppm (t, 2 H) ;
1,35 ppm (s, 9 H) ; 1,1 bis 1,7 ppm (m, 21 H) ; 0,9 ppm (t, 3 H).

**Ansprüche**

1. 4-tert.-Butoxybenzylamine und deren Ammoniumsalze der allg. Formel (I)

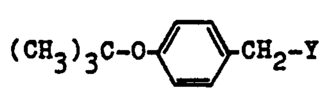

(I)

wobei Y für die Reste

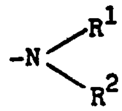 oder

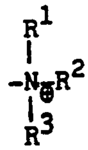 $X^{\ominus}$

steht, in denen $R^1$ und $R^2$ ein Wasserstoffatom, eine Alkyl-, Cycloalkyl-, Aralkyl- oder Arylgruppe mit 1 bis 15 Kohlenstoffatomen, $R^3$ ein Wasserstoffatom, eine Alkyl-, Cycloalkyl oder Aralkylgruppe mit 1 bis 15 Kohlenstoffatomen und $X^{\ominus}$ ein Hydroxylion oder ein Säureanion bedeutet.

2. Verfahren zur Herstellung der Verbindungen gemäß Anspruch 1, dadurch gekennzeichnet, daß man 4-tert.-Butoxybenzaldehyd in Gegenwart eines Hydrierkatalysators mit Wasserstoff und Ammoniak oder einem Amin der Formel (II)

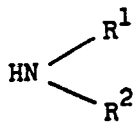 (II)

in der $R^1$ und $R^2$ die vorgenannte Bedeutung besitzen, bei erhöhter Temperatur und erhöhtem Druck umsetzt und gegebenenfalls daraus durch Protonierung mit einer Säure HX bzw. Alkylierung mit einem Alkylierungsmittel $R^3X$, wobei $R^3$ und X die vorgenannte Bedeutung haben, die entsprechenden Ammoniumverbindungen herstellt.

**Claims**

1. A 4-tert.-butoxybenzylamine and its ammonium salts of the general formula (I)

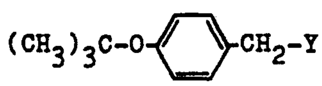 (I)

where Y is

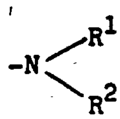 or

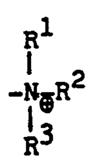 $X^{\ominus}$

in which $R^1$ and $R^2$ are each hydrogen or an alkyl, cycloalkyl, aralkyl or aryl group of 1 to 15 carbon atoms, $R^3$ is hydrogen or an alkyl, cycloalkyl or aralkyl group of 1 to 15 carbon atoms and $X^{\ominus}$ is a hydroxyl ion or an acid anion.

2. A process for the preparation of a compound as claimed in claim 1, wherein 4-tert.-butoxybenzaldehyde is reacted, in the presence of a hydrogenation catalyst, at elevated temperatures and under superatmospheric pressure, with hydrogen and ammonia or an amine of the formula (II)

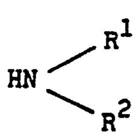 (II)

where $R^1$ and $R^2$ have the above meanings, and, if required, the product is converted to the corresponding ammonium compound by protonation with an acid HX or alkylation with an alkylating agent $R^3X$, where $R^3$ and X have the above meanings.

**Revendications**

1. 4-tertiobutoxybenzylamines et leurs sels ammoniques de formule générale (I)

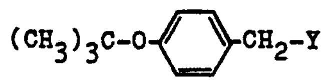

(I)

dans laquelle Y est mis pour les radicaux

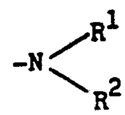

ou

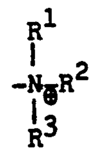

$X^{\ominus}$

$R^1$ et $R^2$ représentant un atome d'hydrogène, un groupe alcoyle, cycloalcoyle, aralcoyle ou aryle contenant 1 à 15 atomes de carbone, $R^3$ un atome d'hydrogène, un groupe alcoyle, cycloalcoyle ou aralcoyle à 1-15 atomes de carbone et $X^{\ominus}$ un ion hydroxyle ou un ion acide.

2. Procédé pour la préparation des composés selon la revendication 1, caractérisé en ce qu'on fait réagir, à haute température et sous pression élevée, un 4-tertiobutoxybenzaldéhyde en présence d'un catalyseur d'hydrogénation avec l'hydrogène et l'ammoniac ou une amine de formule (II)

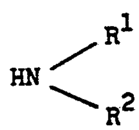

(II)

dans laquelle $R^1$ et $R^2$ ont les significations données ci-dessus et, le cas échéant, on prépare les composés ammoniques correspondants, à partir des produits de la réaction, par protonation avec un acide HX ou alcoylation avec un agent d'alcoylation $R^3X$, $R^3$ et X ayant les significations données ci-dessus.